# DEMANDE DE BREVET EUROPEEN

(11) **EP 0 560 690 A1**
(43) Date de publication de la demande: **15.09.1993**
(21) Numéro de dépôt: 93400653.7
(22) Date de dépôt: 15.03.1993
(51) Int. Cl.: A23L 3/28, A61L 9/01, F25D 13/00

(54) **Dispositif pour l'équipement de chambres froides utilisées pour le stockage de produits alimentaires**

(30) Priorité: 13.03.1992 FR 9203018
(71) Demandeur: Decupper, Jean, F-06400 Cannes (FR)
(72) Inventeur: Decupper, Jean, F-06400 Cannes (FR)
(74) Mandataire: Wagret, Frédéric

(57) **Abrégé**

- Dispositif pour l'équipement de locaux réfrigérés en vue de la conservation de denrées périssables, telles que des chambres froides, et le dispositif est caractérisé en ce qu'il est constitué d'une capacité (3) de structure oblongue pour le traitement de l'air ambiant intérieur audit local, cette capacité (3) communiquant avec l'ambiance du local par une première ouverture (5) constituant une entrée d'air et par une seconde ouverture (5') constituant une sortie d'évacuation de l'air traité et la capacité contient intérieurement au moins une source de rayonnement ultraviolet (2, 2') disposée à l'intérieur du volume de ladite capacité (3) et une source d'ozone (8).

## Description

La présente invention concerne un dispositif utilisable pour l'équipement de locaux soumis à une régulation thermique notamment à une réfrigération en vue de la conservation de denrées périssables tels que des aliments.

Plus spécialement l'invention sera applicable à l'équipement de chambres froides utilisées pour le stockage à court, moyen ou long terme de produits alimentaires de toute nature et notamment des produits carnés.

On connaît le phénomène technique et économique que constitue le développement du stockage de produits alimentaires par le froid. Ce phénomène est bénéfique dans la mesure où il permet de réguler la rencontre de l'offre et de la demande dans le cas notamment de productions saisonnières ou de productions trop sensibles à l'évolution des cours.

L'abondance de certaines productions agricoles excédentaires notamment dans les pays fortement industrialisés doit également être résorbée au moins momentanément par une mise en stock des produits.

De plus, les locaux réfrigérés sont aujourd'hui couramment utilisés par les professionnels pour le stockage à court et moyen terme notamment de produits carnés permettant de constituer des stocks tampons dans le cadre des activités agroalimentaires ou pour fournir des équipements de restauration collective.

On sait que les dispositifs du type "chambre froide" doivent subir un cycle thermique parfaitement ajusté en fonction de la nature des produits en vue d'éviter la prolifération, au dessus d'une certaine température, de germes pathologiques.

Cependant les conditions de stockage ainsi imposées aux produits carnés ne sont pas favorables à une bonne conservation des qualités organolepti- ques du produit et au maintien d'une apparence satisfaisante pour le consommateur.

On sait en effet que la température de conservation, notamment de viande, qui est nécessaire pour l'obtention de conservation correspondante aux normes sanitaires, aboutit à une déshydratation du produit et à un noircissement superficiel.

Ces inconvénients restent relativement mineurs mais sont généralement peu appréciés du consommateur qui aime à retrouver sur l'étal du boucher une viande présentant la belle apparence rouge de la viande fraîche.

De plus, on sait que la présence de la viande conservée dans ces conditions développe des composés volatils qui entraînent en permanence dans le milieu fermé ainsi créé des odeurs parasites qui sans être désagréables ne correspondent cependant pas à une image de qualité et notamment à une sensation olfactive associée à la viande fraîche ; la présence de ces odeurs secondaires risquant de pénétrer, ne serait-ce que superficiellement, les produits représente par conséquent un inconvénient auquel il n'a pas été porté remède à ce jour.

On a sans doute depuis longtemps utilisé des tubes émetteurs d'un rayonnement ultraviolet pour assainir l'atmosphère d'un milieu notamment dans les points de vente et de distribution de produits alimentaires.

Mais cette action sur les germes reste totalement inefficace vis-à-vis des odeurs.

On connaît également l'utilisation de l'ozone pour assainir et purifier l'ambiance de milieux confinés, notamment les réseaux de chemin de fer urbains souterrains.

L'invention vise à réaliser une combinaison sy- nergétique entre les deux moyens ainsi conjugués en vue de permettre la réalisation d'un appareil unique susceptible d'apporter aux équipements de réfrigération en milieux confinés notamment dans les chambres froides à la fois un effet positif par l'élimination des germes et le maintien d'un milieu sensiblement stérile tout en éliminant parallèlement les odeurs parasites susceptibles de se développer dans ce milieu.

A cet effet l'invention concerne un dispositif pour l'équipement de locaux réfrigérés en vue de la conservation de denrées périssables, telles que des chambres froides, et le dispositif est caractérisé en ce qu'il est constitué d'une capacité de structure oblongue pour le traitement de l'air ambiant intérieur audit local, cette capacité communiquant avec l'ambiance du local par une première ouverture constituant une entrée d'air et par une seconde ouverture constituant une sortie d'évacuation de l'air traité et la capacité contient intérieurement au moins une source de rayonnement ultraviolet disposée à l'intérieur du volume de ladite capacité et une source d'ozone.

De préférence la source d'ozone est constituée par un générateur d'ozone de type connu disposé à la sortie de ladite capacité et apte à mélanger à l'air traité un pourcentage d'ozone en quantité déterminée.

Plus spécialement encore l'entrée de la capacité comporte des moyens de mise en circulation de l'air ainsi aspiré depuis le milieu intérieur au local et pulsé vers le volume intérieur de la capacité.

Selon encore une autre caractéristique, l'entrée est constituée d'un couloir de section inférieure à la section de passage du volume intérieur de ladite capacité contenant le tube ultraviolet, et de telle sorte que la vitesse de l'air circulant dans le passage d'entrée est supérieure à la vitesse de l'air circulant à l'intérieur du volume de ladite capacité.

Selon une autre caractéristique de l'invention, la section du passage constituant la sortie pour l'évacuation de l'air traité est sensiblement équivalente à la section du passage constituant l'entrée vers la capacité intérieure ; de sorte que la vitesse de l'air circulant dans ce passage de sortie soit sensiblement égale à la vitesse de l'air circulant dans le passage d'entrée.

Selon une autre caractéristique, la source de rayonnement ultraviolet est constituée par un tube de type connu émettant un rayonnement ultraviolet bactéricide correspondant à la raie d'émission de 2.537 angstrôm.

Et selon une autre caractéristique plus avantageuse, la capacité comporte intérieurement une pluralité de tubes ultraviolets disposés en deux points opposés sensiblement à poximité des parois de ladite capacité, l'air traité circulant ainsi entre les deux tubes.

Selon une autre caractéristique, le dispositif comporte des moyens de programmation aptes à déclencher, selon un cycle déterminé et programmé, la mise en service respectivement des moyens de circulation de l'air et l'alimentation des tubes ultraviolets en énergie électrique et l'émission d'ozone.

Selon encore une autre caractéristique, les parois intérieures de la capacité comportent des inégalités de surface, telles que des ondulations, ainsi propres à créer des turbulences au sein du milieu gazeux avec une répartition aléatoire des filets d'air en multipliant ainsi les chances pour les poussières microscopiques véhiculées dans l'air d'être exposées sur leurs diverses faces au rayonnement ultraviolet en assurant ainsi la destruction des germes portés par lesdites poussières par suite de l'action bactéricide du rayonnement.

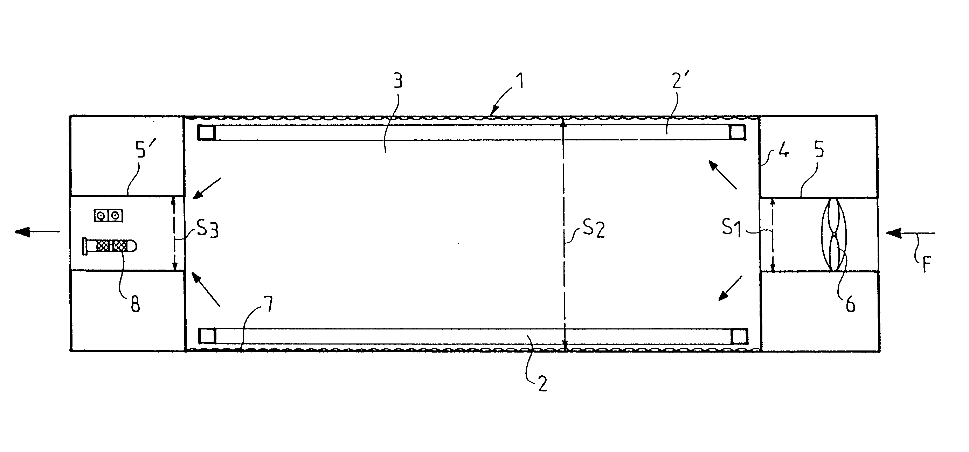

D'autres caractéristiques et avantages de l'invention ressortiront de la description qui suit et qui est donnée en rapport avec une forme de réalisation particulière présentée à titre d'exemple non limitatif et en se référant au dessin annexé.

La Figure unique représente une vue en coupe d'un dispositif réalisé conformément à l'invention et propre à équiper notamment une chambre froide en vue d'assurer un effet à la fois bactéricide et d'épuration et de désodorisation de l'air.

Selon la Figure unique on voit que l'on a prévu un appareil constitué d'une capacité définie par une cage 1 ici de forme générale cylindrique dans laquelle sont disposés, diamétralement opposés, les deux tubes ultraviolets respectivement 2 et 2'.

La cage définissant la capacité 3 est de forme oblongue, le cylindre ayant une longueur supérieure à 3 à 4 fois le diamètre.

Le cylindre est refermé à ses deux extrémités par les parois transversales 4 et 4', dans la partie centrale, desquelles débouchent les passages ou couloirs cylindriques respectivement 5 et 5'.

Le couloir 5 constitue le couloir d'entrée et il est muni à cet effet d'un circulateur ici constitué de l'hélice d'un ventilateur 6.

L'air pulsé est ainsi aspiré depuis l'extérieur selon la flèche F et il se dirige vers la capacité intérieure 3.

On voit que la section S1 du passage 5 est sensiblement inférieure à la section S2 qui correspond au diamètre intérieur de la capacité 3.

Dans ces conditions l'air aspiré mis en circulation par le ventilateur 6 traverse le passage 5 à une vitesse déterminée V1 largement supérieure à la vitesse V2 de circulation de l'air au sein de la capacité 3.

Dans ces conditions l'air subit un effet "venturi" provoquant un ralentissement immédiatement après la section resserrée 5 de sorte que l'air circule lentement à l'intérieur de la capacité 3 depuis le passage d'entrée 5 vers le passage de sortie 5'.

Et pendant son temps de séjour, qui peut être ajusté et régulé aisément, par la vitesse de mise en circulation du ventilateur6, l'air se trouve ainsi exposé à l'action bactéricide du rayonnement provenant des tubes 2 et 2'.

La paroi intérieure de la capacité comporte des inégalités de surface constituées par exemple des ondulations 7 qui vont éviter que les filets d'air suivent un régime régulier lent et laminaire ; ces inégalités de surface vont au contraire entraîner des turbulences et un régime de circulation tourbillonnaire au sein de la capacité 3.

Dans ces conditions, les filets d'air circulant en tourbillons entraînent avec eux les particules, notamment les poussières porteuses de germes et bactéries, et ces derniers vont se trouver ainsi exposés sur toutes les faces aux rayonnements linéaires et rectilignes provenant des deux tubes situés dans deux zones diamétralement opposées de la capacité 3.

Le passage de sortie 5' est quant à lui d'une section sensiblement équivalente au passage d'entrée 5, de sorte que la vitesse de circulation au sein de ce passage V3 sera équivalente à la vitesse de circulation VI qui règne au sein du passage d'entrée 5.

On peut ainsi constater que la vitesse de sortie étant égale à la vitesse d'entrée, on évite de créer au sein du milieu intérieur à la chambre (à l'extérieur de la capacité) des remous ou tourbillons qui viendraient perturber la stabilité thermique de l'ensemble et notamment qui risqueraient de créer un dessèchement des produits situés à proximité de l'appareil.

Le passage 5' comporte un générateur d'ozone 8 qui permet ainsi de mêler à l'air traité une quantité programmée d'ozone en apportant au milieu ainsi traité l'effet de purification et de stabilisation de l'air notamment sur le plan organoleptique en détruisant les odeurs parasites.

On sait en effet que les odeurs régnant dans un milieu sont constituées de composés extrêmement volatils de nature organique qui, exposés à l'action de l'ozone, oxydant puissant, sont automatiquement détruits.

Un programmateur 9 incorporé dans l'ensemble permet de réguler selon un cycle déterminé la mise en fonction des divers moyens conjugués et combinés au sein de l'appareil ; le programmateur peut ainsi en fonction des besoins déterminer les cycles de mise en fonctionnement conjugués ou séparés de l'ensemble et notamment l'alimentation électrique du ventilateur 6, des tubes ultraviolets 2 et 2'et du générateur d'ozone 8.

L'appareil selon l'invention permet une meilleure conservation des denrées.

D'une part, il évite l'incorporation d'odeurs secondaires ou parasites aux produits et par conséquent procure une meilleure approche olfactive du produit.

Il assure par ailleurs des conditions de conservation améliorées dans la mesure où il évite une perte de poids, notamment des produits carnés, et également il annihile ou limite considérablement le phénomène de coloration.

On a constaté en effet que grâce à l'utilisation des moyens de l'invention qui ajoutent à l'effet du froid un effet de stérilisation du milieu et de suppression des odeurs parasites, il était possible de remonter de 2 à 3° la température de conservation, de sorte que l'on limite dans ces conditions le phénomène d'évaporation et par conséquent de dessèchement avec la conséquence économiquement domageable d'une perte de poids, indépendamment d'une détérioration des qualités alimentaires et gustatives du produit.

L'appareil selon l'invention permet encore une économie d'énergie précisément en raison des conditions thermiques plus élevées qui peuvent être supportées par la chambre froide tout en assurant des conditions parfaites de conservation.

Il suit que la déperdition des frigories au sein du milieu est ainsi réduite en permettant de limiter par conséquent la consommation électrique.

Le dispositif de l'invention ne représente aucune menace pour le personnel dans la mesure où on évite l'émission d'un rayonnement d'ultraviolet susceptible d'atteindre le personnel, les tubes étant enfermés dans la capacité où l'airest amené en circulation pour son traitement régulier.

L'appareil peut donc être maintenu en action malgré les allées et venues des personnes livrant ou délivrant des produits au sein de la chambre froide.

Enfin le générateur d'ozone apporte son effet vi- rulicide en augmentant l'action de décontamination apportée par les ultraviolets ; l'ozone améliore donc les conditions de travail du personnel affecté au fonctionnement et à l'entretien de la chambre froide, outre l'apport bénéfique constaté dans la présentation des produits au terme de leur période de conservation.

Selon une forme de réalisation les dimensions de la capacité 3 sont, en centimètres : L = 97, P = 20 et A = 30 soit un volume de l'ordre de 60 dm3.

Pour un débit de ventilation de 80 m3/H la vitesse volumique est de l'ordre de 2,6 secondes. Elle sera de préférence comprise entre 1 et 10 secondes.

L'appareil est destiné à traiter l'air d'un local, qui, hormis les entrées et sorties de personnel, ainsi que la climatisation ou réfrigération, n'occasionne qu'un faible taux de renouvellement de l'air de l'enceinte.

Si l'on prend comme base qu'un appareil est à même de traiter un local d'environ 50 m3 dans de bonnes conditions, cela signifie que la quasi totalité de l'air de l'enceinte du local passera par l'appareil en 40 minutes environ, soit en fonctionnement continu, 36 fois en 24 heures.

Cette notion de traitement continu, pour compenser les apports extérieurs de bactéries introduites par l'ouverture des portes, entre autre après avoir traité les éléments présents au moment de la mise en route, fait apparaître ou ressortir l'efficacité de l'appareil.

On utilisera de préférence un appareil pour un volume de l'enceinte traité inférieur à 50 m3 et deux appareils pour une enceinte entre 50 et 100 m3.

## Revendications

1 - Dispositif pour l'équipement de locaux réfrigérés en vue de la conservation de denrées périssables, telles que des chambres froides, et le dispositif est caractérisé en ce qu'il est constitué d'une capacité (3) de structure oblongue pour le traitement de l'air ambiant intérieur audit local, cette capacité (3) communiquant avec l'ambiance du local par une première ouverture (5) constituant une entrée d'air et par une seconde ouverture (5') constituant une sortie d'évacuation de l'air traité et la capacité contient intérieurement au moins une source de rayonnement ultraviolet (2, 2') disposée à l'intérieur du volume de ladite capacité (3) et une source d'ozone (8).

2 - Dispositif selon la revendication 1,
et caractérisé en ce que la source d'ozone est constituée par un générateur d'ozone (8) de type connu disposé à la sortie (5') de ladite capacité et apte à mélanger à l'air traité un pourcentage d'ozone en quantité déterminée.

3 - Dispositif selon l'une des revendications 1 ou 2,
et caractérisé en ce que l'entrée de la capacité (3) comporte des moyens de mise en circulation de l'air (6) ainsi aspiré depuis le milieu intérieur au local et pulsé vers le volume intérieur de la capacité (3).

4 - Dispositif selon l'une des revendications 1, 2 ou 3,
et caractérisé en ce que l'entrée (5) est constituée d'un couloir de section (S^{l}) inférieure à la section (S²) de passage du volume intérieur de ladite capacité (3) contenant la source de rayonnement ultraviolet (2), et de telle sorte que la vitesse de l'air circulant dans le passage d'entrée est supérieure à la vitesse de l'air circulant à l'intérieur du volume de ladite capacité.

5 - Dispositif selon l'une des revendications 1 à 4,
et caractérisé en ce que la section (S³) du passage constituant la sortie (5') pour l'évacuation de l'air traité est sensiblement équivalente à la section (S^{l}) du passage constituant l'entrée (5) vers la capacité intérieure (3) ; de sorte que la vitesse de l'air circulant dans ce passage de sortie soit égale à la vitesse de l'air circulant dans le passage d'entrée.

6 - Dispositif selon l'une des revendications 1 à 5, et caractérisé en ce que la source de rayonnement ultraviolet est constituée par un tube de type (2, 2') connu émettant un rayonnement ultraviolet bactéricide correspondant à la raie d'émission de 2.537 angstrôm.

7 - Dispositif selon la revendication 6,
et caractérisé en ce que la capacité (3) comporte intérieurement une pluralité de tubes (2, 2') ultraviolets disposés en deux points opposés sensiblement à proximité des parois de ladite capacité, l'air traité circulant ainsi entre les deux tubes.

8 - Dispositif selon l'une des revendications 1 à 7, et caractérisé en ce qu'il comporte des moyens de programmation (9) aptes à déclencher, selon un cycle déterminé et programmé, la mise en service de l'émission d'ozone (8).

9 - Dispositif selon l'une des revendications 1 à 8, et caractérisé en ce que les parois intérieures de la capacité (3) comportent des inégalités de surface (7), telles que des ondulations, ainsi propres à créer des turbulences au sein du milieu gazeux avec une répartition aléatoire des filets d'air en multipliant ainsi les chances pour les poussières microscopiques véhiculées dans l'air d'être exposées sur leurs diverses faces au rayonnement ultraviolet en assurant ainsi la destruction des germes portés par lesdites poussières par suite de l'action bactéricide du rayonnement.

10 - Dispositif selon l'une des revendications 1 à 9,
caractérisé en ce que le temps de contact de l'air traité dans l'appareil est compris entre 1 et 10 secondes.
